# EUROPEAN PATENT APPLICATION

(11) **EP 1 407 736 A1**
(43) Date of publication of application: **14.04.2004**
(21) Application number: 02726447.2
(22) Date of filing: 21.05.2002
(51) Int. Cl.: A61F 13/15

(54) **INTERLABIAL PAD INDIVIDUAL PACKAGING VESSEL, AND INDIVIDUAL PACKAGING BODY**

(30) Priority: 22.05.2001 JP 2001152403; 05.03.2002 JP 2002108594
(71) Applicant: UNI-CHARM CORPORATION, Kawanoe-shi Ehime 799-0111 (JP)
(72) Inventor: MIZUTANI, Satoshi, Uni-Charm Corporation, Mitoyo-gun, Kagawa 769-1602 (JP); YAMAKI, Koichi, Uni-Charm Corporation, Mitoyo-gun, Kagawa 769-1602 (JP); NODA, Yuki, Uni-Charm Corporation, Mitoyo-gun, Kagawa 769-1602 (JP); TOKUMOTO, Megumi, Uni-Charm Corporation, Mitoyo-gun, Kagawa 769-1602 (JP); SAKAI, Akane, Uni-Charm Corporation, Mitoyo-gun, Kagawa 769-1602 (JP)
(74) Representative: Purdy, Hugh Barry
(86) International application number: PCT/JP2002/004890
(87) International publication number: WO 2002/094154

(57) **Abstract**

The present invention relates to a wrapping container for individually wrapping an interlabial pad that is easy to be unwrapped and a wrapping body comprising this wrapping container to provide an interlabial pad individual wrapping container including an interlabial pad within a wrapping container that makes it possible to smoothly take out the interlabial pad included therein from the wrapping container.

The unwrapping operation is made simple by maintaining a region (7) where the wearer holds a wrapping container (3) for unwrapping the wrapping container (3). Moreover, an individual wrapping body (1) is provided to allow the wrapping container (3) and the interlabial pad to be simultaneously held so that the interlabial pad included therein is also indirectly held when holding the wrapping body (1), thereby allowing sanitary unwrapping operation.

## Description

### Background of the Invention

### Technical field

The present invention relates to a wrapping container for individual interlabial pad wrapping with the feature of easy unwrapping operation and a wrapping body comprising the wrapping container.

### Background Art

Conventionally, a sanitary napkin and a tampon are used generally as sanitary products for female. Here, there have been great efforts to prevent the leak of menstrual blood from the gap caused by poor adhesion near the ostium vaginae as for the sanitary napkin. Moreover, as for the tampon, there have been great efforts for relieving the foreign feeling and the discomfort when wearing the tampon and intervaginal wearing trouble due to the nature of those products.

Under such situation, sanitary products of the interlabial pad have attracted people as a sanitary product positioned between the sanitary napkin and the tampon in recent years.

The interlabial pad is worn by inserting it between the labia, being characterised in that it is difficult to cause the leak of menstrual blood because of higher adhesion to the body than that of the sanitary napkin and that psychological resistance thereof on wearing is lower than that of the tampon which is inserted into the vagina.

Here, for such interlabial pad, there are insufficient development and provision of members used for packaging it.

For this subject, it is favorable to be able to rapidly and easily perform a series of operation in which a wrapping container containing the interlabial pad is unwrapped and the interlabial pad is taken out because the interlabial pad is worn under hectic conditions.

Moreover, the sanitary napkin and panty-liner which are other generally used sanitary products are applied with adhesive on a face of garment to be fixed to the garment such as an underwear, and the applied adhesive generally contacts a non-stick processed inner surface of the wrapping material directly or a non-stick processed sheet by folding the product with the inserted non-stick processed sheet. Thus, the product is generally provided with the wrapping container enclosing it with the wrapping material.

In contrast with this, the interlabial pad is fixed by inserting it between labia so that it is a common practice that there is no adhesive applied to the opposite side face to the body side face, and the interlabial product and wrapping material are not packaged as one unit so that, when the wrapping container is unwrapped, the product is easily dropped from the wrapping container due to the impact, thereby rapid wearing is prevented because a good care should be taken not to cause such incident in unwrapping operation.

### Disclosure of the Invention

The present invention is made to solve such task, and it is the purpose to provide an interlabial pad individual wrapping body including an interlabial pad in a wrapping container which makes it possible to smoothly take out the interlabial pad from the wrapping container containing the interlabial pad.

The present invention is characterized in that, in order to solve such task, the unwrapping operation is made simple by maintaining a region where a wearer holds the wrapping container so as to unwrap the wrapping container.

Furthermore, the present invention is characterized in that an individual wrapping body is provided which allows sanitary unwrapping operation by making the wrapping container and the interlabial pad be grabbed at the same time so that the contained interlabial pad is indirectly held when the wrapping body is held.

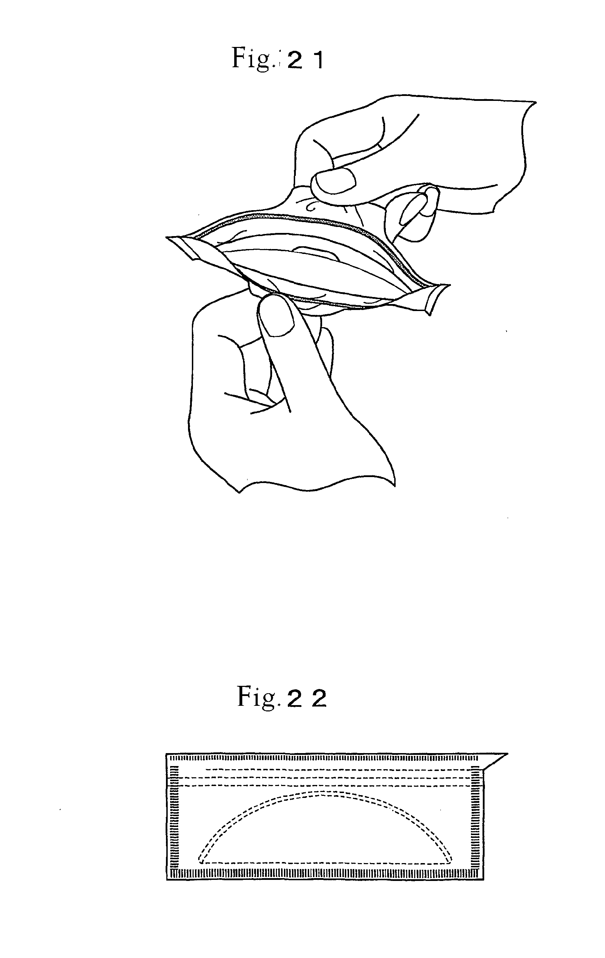

More concretely, the present invention provides followings:
(1) An individual wrapping container for wrapping individually an interlabial pad to be held between labia, comprising: a main body containing said interlabial pad and a cover that covers a part of said main body the cover forming an unwrapping portion at an end edge portion to be unwrapped to open the individual wrapping container, wherein said cover is shaped to have a cut off portion at a place where said individual wrapping container is held when the individual wrapping container is opened, and wherein the individual wrapping container has a predetermined region where a finger of a wearer is are applied.
   According to the present invention, in the individual wrapping container packaging an unused interlabial pad (hereafter, called simply "wrapping container"), a finger of a wearer holding the wrapping body does not obstruct this unwrapping operation when the wrapping container is unwrapped by opening a comer which covers a main body containing the interlabial pad. That is to say, the cover is provided with a shape that avoids the finger applied to the main body so that the wearer does not press the cover with the finger holding the wrapping container. Therefore, the wearer can unwrap the wrapping container in a state where the wearer holds the wrapping body at the beginning thereof, namely, unwrapping the package without re-holding the wrapping body, making it possible to rapidly and smoothly unwrap the wrapping container.
(2) An individual wrapping container according to (1), characterized in that said end edge portion of said cover is inflected or inclined to a bottom side of the individual wrapping container.
   According to the present invention, the edge of the cover is curved or inclined beforehand in a individual wrapping container wrapping an unused interlabial pad (hereafter, called simply "wrapping container"). Therefore, it is made possible to direct the wearer to hold the wrapping body by applying fingers on the region where there is no cover when the wearer holds the wrapping body.
   In the case where the edge of the cover is curved, the part where the finger of the wearer is applied is hollowed out to curve the shape or it is possible that the center of the end of the edge is projected in a shape of M and other part is curved toward the sealed opening of the interlabial pad. By doing this, it is possible to unwrap the wrapping container by holding said projected portion when unwrapping the wrapping container. Moreover, it is possible to more quickly unwrap the container because it is possible to instantly identify this part where the wearer unwraps the cover by holding with a finger. Moreover, because there are two portions where the wearer may hold the wrapping body without applying the finger to the cover, it is possible to secure the rapid unwrapping operation whether the dexterous hand of the wearer is right or left.
   On the other hand, in the case where the end edge portion of the cover is inclined, the exposure ratio is gradually diminishing from one side on to the other side of the wrapping container on the surface where the cover and the main body and an unwrapping portions are formed. Therefore, it is possible to unwrap the cover as it is, since the more exposed surface may be held by the wearer.
(3) An individual wrapping container for wrapping individually an interlabial pad to be held between labia, comprising: a main body containing said interlabial pad and a cover that covers a part of said main body, the cover forming an unwrapping portion at an end edge portion to be unwrapped to open the individual wrapping container, wherein a surface of said main body has an instruction to show where said individual wrapping container is held when the individual wrapping container is unwrapped.
   According to the present invention, a position where the wearer holds the wrapping body is clearly indicated to unwrap the individual wrapping container (hereafter, called simply " wrapping container"). Therefore, the wearer can hold the wrapping body by applying a finger naturally on a position where is suitable for unwrapping operation without pondering.
(4) An individual wrapping container according to (3), characterized in that said instruction is made visual or touchable.
   According to the present invention, a position where the wearer holds the wrapping body is made clearly found by a glimpse or touch.
   As such products, it is possible to adopt a method to apply an illustration or pattern of a shape of finger or perform an emboss processing on the position where a finger is applied.
(5) An individual wrapping container for wrapping individually an interlabial pad to be held between labia, comprising: a main body containing said interlabial pad and a cover which overlaps on a part of the main body, the cover having an end edge portion forming an unwrapping portion to unwrap said individual wrapping container, wherein the main body has a fine projection at least on an internal surface of said main body.
   According to the present invention, fine projections are provided on the inside of the individual wrapping container (hereafter, called simply "wrapping container"). Therefore, when an used interlabial pad is accommodated in the wrapping container which an unused interlabial pad has been taken out, menstrual blood is prevented from adhering on the inside of the wrapping container, thereby making an appearance of the wrapping container clean when it is thrown away.
(6) An individual wrapping container according to any one from (1) to (5), wherein said individual wrapping container comprises a container on which a series of sheets for wrapping is wound and wherein said cover comprises an overlapping portion that is formed by overlapping both ends of a wrapping sheet and a vicinity of said both ends, wherein said unwrapping portion comprises one end of said wrapping sheet, and wherein said individual wrapping container is unwrapped by unfolding said overlapping portion from said unwrapping portion.
   According to the present invention, an individual wrapping container (hereafter, called simply " wrapping container") is formed with one sheet and the interlabial pad is wrapped by winding the pad with the sheet to shape the wrapping body. Then, one end portion of said sheet overlaps other part of the sheet on one side of the wrapping container so that the one end portion of the sheet forms an unwrapping portion of the wrapping container. The one end portion on of the sheet positioned on the upper surface of the overlapped portion and a vicinity portion thereof form the cover. And the part other than the overlapped portion is to be a holding region where the wearer holds the wrapping body.
   In the present invention, the wrapping container comprises one sheet as a wrapping material so that the manufacturing process is made easy as compared with the case where the interlabial pad is wrapped by using a plurality of wrapping materials. Moreover, since a process to close the whole peripheral edge portions of a plurality of wrapping materials during manufacturing the container is eliminated, in enclosing the interlabial pad in a wrapping container, it is less likely to produce a gap between the circumferencial edge of the interlabial pad and the wrapping container, so as to make the whole wrapping body compact, and enhance portability of the body.
(7) An individual wrapping container according to (6), comprising: an unwrapping portion for sealing the interlabial pad, and a bottom line on the opposite side; wherein the dimension of the main body becomes smaller as it proceeds from said bottom line to said unwrapping portion.
   According to the present invention, the longitudinal dimension of the opening is made shorter than the longitudinal dimension of the base in the main body of the individual wrapping container (hereafter, called simply "wrapping container"). Namely, the ratio between the length in unwrapping direction of the main body of the wrapping container and the length of the opening is made smaller than the ratio between the length in unwrapping direction of the main body and the length of the base. Therefore, the dimension between both sides of the main body will be made narrow in the opening portion so that it is made easier for the wearer to transmit the force in unwrapping direction which is exerted by the wearer's fingers holding the cover in-between for unwrapping to both end portions of the opening portion. As a result, when unwrapping the wrapping body by unfolding the wrapping container into a sheet, it is made possible to shorten the time to peel the bonded portion on both sides of the main body so that it is also made possible to shorten the unwrapping time of the wrapping container, thereby making the unwrapping operation more rapid and easier.
(8) An individual wrapping container according to any one from (1) to (7), wherein said individual wrapping container is provided with a re-sealable sealing means on a portion where said cover and said main body are brought into contact.
   According to the present invention, a re-sealable sealing means is provided on the part where the individual wrapping container (hereafter, called simply "wrapping container") is unwrapped. Therefore, it is possible to seal the used interlabial pad in the wrapping container by fastening the unwrapping portion of the wrapping container with such sealing means when the wrapping container containing the used interlabial pad sealed therein is disposed. Therefore, it is possible to prevent such risk that, while the used interlabial pad that has been in the wrapping body is discarded of in a sanitary container, the interlabial pad is unfolded to stain the fingers with oozing menstrual blood that the interlabial pad sticks out of the wrapping container to stain the hand with the menstrual blood, and that further, only the interlabial pad is dropped onto the floor. Thereby, it is possible to discard the used interlabial pad in a sanitary container in a sanitary condition.
   It is possible to easily recognize the unwrapping portion where the wearer pinches instantly the container with fingers if such sealing means has a color or a printed illustration. Thereby, the unwrapping operation becomes smoother.
   There is, for example, a belt-shaped member on whose one surface re-peelable adhesive is applied as said sealing means, concretely, it is possible to use a widely known members such as a film layer whose one surface is applied with adhesive or a tissue whose one side is applied with adhesive if flushing characteristics are considered. As other sealing means, it is conceivable to attach a MAGIC TAPE (Japanese Registered trademark) or to attach a fastener which engages the male part and female part each other.
   In the case where no such belt-shaped member is provided, it is possible to make it difficult for dust to enter from the unwrapping portion when carrying by applying adhesive continuously or intermittently on the edge of the cover, thereby allowing the interlabial pad to be sanitarily kept. Moreover, applying adhesive on the position shifting 1 to 10 mm in unwrapping direction provides a dry edge where there is no adhesive applied to the unwrapping opening so that it is possible to easily unwrap the wrapping container by picking such dry edge. The adhesive is selected from re-peelable hot-melt type adhesive without limitation and applied with known application pattern such as plane-shape, stripe-shape, and helical shape or dotted shape.
(9) An individual wrapping container according to (8), characterized in that said sealing means has a dry edge and that said dry edge projects from the bottom side of said individual wrapping container.
   According to the present invention, one end of the sealing means provided on the cover of the wrapping container is a dry edge where no adhesive is applied, and this dry edge projects from the base of the wrapping container, being completely separated from the wrapping material. Therefore, the wearer can not only easily recognize the position to be pinched by fingers for unwrapping the wrapping body but also easily pull the dry edge portion in unwrapping direction by pinching it with fingers. As a result, it is made possible to shorten the unwrapping time of the wrapping container, Thereby, it is possible to make unwrapping operation faster and easier.
(10) A wrapping body, comprising an interlabial pad to be held between labia and an individual wrapping container for individual wrapping said interlabial pad, wherein the interlabial pad wrapped in said interlabial container constitutes the wrapping body comprising said interlabial pad wrapped in said individual wrapping container, and wherein the interlabial pad in said individual wrapping container is arranged on a position where at least one part of said interlabial pad is held at the same time when holding said individual wrapping container is held.
   According to the present invention, it is possible to simultaneously hold the individual wrapping container (hereafter, called simply "wrapping container") and the interlabial pad included therein with holding fingers when the wrapping container is held. Therefore, it is possible to prevent the included interlabial pad from springing out due to the unwrapping impact even if the unwrapping portion is pinched with the hand opposite to the hand holding the wrapping body to unwrap the wrapping container. Thereby, unwrapping operation may be made in a sanitary condition.
   With this respect, a wrapping container is disclosed in US Patent No. 6131736 wherein, in order to provide a separate sack, a series of sheet is folded, one inner surface is faced with the other inner surface with this folded part as an axis, further, the circumference other than folded part is sealed, and, at least, the sealed one side is made a re-sealable portion as a taking-in and -out opening for the interlabial pad. According to this wrapping container, it is possible to take out the interlabial pad without touching the surface where the interlabial product contacts with body when wearing it so that the interlabial product can be inserted between labia in sanitary state.
   However, in such conventional example, a part near unwrapping opening is pinched with each finger of both hands to peel the re-sealing portion of the sealing opening so that the interlabial product included in the wrapping container is not fixed with fingers via wrapping container when unwrapping it as shown in Fig. 21. Therefore, there is quite a high risk that the included interlabial product may spring out from the wrapping container due to the impact of unwrapping so that the interlabial product may contact fingers and drop onto the floor, resulting in making the interlabial product dirty.
   As shown in Fig. 22, also in the wrapping container wherein perforation is provided and, then, torn for unwrapping, some wearer may pinch the wrapping body with fingers of both hands on one side and the other side across the perforation near the unwrapping opening in order to quickly exchange a new interlabial product so as to easily tear the perforation with enhanced power transmission of the fingers. However, there is a risk that the interlabial product may spring out from the wrapping container due to the impact of unwrapping because the interlabial product is not pinched by the fingers over the container.
   In contrast to this, the wrapping body relating to the present invention includes the interlabial pad in the wrapping container so that the interlabial pad is also held via the wrapping container when the wrapping container is held. Thereby, it is possible for the wearer to take out the interlabial pad in a sanitary condition without a special caution.
   Moreover, in the present invention, the method to unwrap the wrapping container is not limited if the interlabial pad is positioned in the wrapping container so that the interlabial pad does not spring out when it is unwrapped. It is also possible to provide, for example, a perforation having cut and non-cut portions aligned alternatively, or a notch on the unwrapping part of the unwrapping portion, namely, the unwrapping opening to initiate tearing the individual wrapping container.
   In the case where the unwrapping portion is formed by perforation, it is preferable that the cut portion is in the range of 0.5 to 5 mm in length and maximum of 3 mm in width, and the non-cut portion is in the range of 0.5 to 3 mm in length. Thus, it is possible to guide the tearing direction made by the wearer not to swerve from the unwrapping portion, and to prevent dust from entering from the unwrapping portion to the inside of the individual wrapping container.
   It is desirable that the unwrapping portion has the perforation with the cut portion piercing the thickness of the individual wrapping container in order to make it easier to tear it.
(11) An individual wrapping body according to (10), wherein said individual wrapping container is the individual wrapping container as recited in any one from (1) to (9).
   According to the present invention, the interlabial pad is included in the individual wrapping container, etc. that allows rapid unwrapping operation. Therefore, the unwrapping operation is made quicker and smoother.
(12) A wrapping body according to (11), wherein said interlabial pad and said individual wrapping container have a longitudinal direction and a lateral direction and substantially form an elongated shape in the longitudinal direction, a dimension in the longitudinal direction of said individual wrapping container is in a range of 105 to 130% of a dimension in the longitudinal direction of said interlabial pad.
   According to the present invention, the dimension in longitudinal direction of the individual wrapping container (hereafter, called simply "wrapping container") is set a dimension wherein the interlabial pad does not play unnecessarily within the wrapping container as compared to the dimension in longitudinal direction of the interlabial pad included therein.
   Here, if the dimension of the container is larger than the above-mentioned dimension range, it may be more difficult to place the interlabial pad near the unwrapping portion. The interlabial pad may not be indirectly pinched with fingers over the wrapping material when the wrapping body is held by the fingers. Thereby it may be more likely that the contained interlabial pad springs out due to the impact of unwrapping.
   On the other hand, if the dimension of the container is smaller than the above-mentioned range, there is such a risk of damaging the interlabial pad such as tearing the contained pad with the unwrapping portion when the unwrapping the unwrapping opening is unwrapped. Moreover, there is insufficient space to take out the interlabial pad from the wrapping container, so that it may be difficult to take out the interlabial pad smoothly and that the unwrapping operation may become troublesome.
   With this respect, it is possible to conduct a series of operation such as rapid and easy unwrapping of the wrapping container and take-out of the interlabial pad without said inconvenience by setting the dimension according to the present invention.
(13) A wrapping body according to (10) or (11), wherein the dimension in said lateral direction of said individual wrapping container is set in a range of 105 -- 130% of the dimension in the lateral direction of said interlabial pad.
   According to the present invention, the dimension in the lateral direction of the individual wrapping container (hereafter, called simply "wrapping container") is set in the range of 105 to 130% of the dimension in the longitudinal direction of the interlabial pad.
   Here, if the dimension of the container is larger than the above-mentioned dimension range, it is difficult to place the interlabial pad near the unwrapping portion so that it may be impossible to indirectly pinch the contained interlabial pad via the wrapping material when the wrapping body is held with fingers. Thus, the risk that the contained interlabial pad springs out due to the impact of unwrapping may be increased.
   On the other hand, if the dimension of the container is smaller than the above-mentioned range, there is such a risk of damaging the interlabial pad such as tearing the contained interlabial pad at the same time when the unwrapping opening is unwrapped. Moreover, there is insufficient space to take out the interlabial pad from the wrapping container sc that it is difficult to take out the interlabial pad smoothly and that the unwrapping operation may be troublesome.
   With this respect, it is possible to conduct a series of operation such as rapid and easy unwrapping of the wrapping container and take-out of the interlabial pad without said inconvenience by setting the dimension according to the present invention.
(14) An individual wrapping container for individually wrapping an interlabial pad to be held between labia, comprising a main body containing said interlabial pad and a cover which overlaps a part of said main body, the cover having an end edge portion forming an unwrapping part to unwrap the individual wrapping container, wherein said cover and said main body are lightly bonded each other at both end portions in a perpendicular direction to an unwrapping direction of the individual wrapping container, and wherein said cover has an angular shape extending toward both sides of said main body from one part of said unwrap part as a vertex.
   Here, the "angular shape" may be a shape having some angle in some way, but not necessarily having a strict "angle." The shape includes variations such as a shape having a side edge in a curved line.
   Moreover, "lightly bonded" means that it is bonded in a state wherein it is possible to be peeled with operation by a female without difficulty. In the individual wrapping container according to the present invention, a female without difficulty can peel the cover off from the main body.
   According to the configuration in (14), the unwrapping part is in an "angle" with a specific angle, but not in a straight line so that it is easy to pinch the unwrapping part of the cover, and that it is possible to easily pull up the cover overlapping the main body.
(15) An individual wrapping container for individually wrapping an interlabial pad to be held between labia, comprising: a main body containing said interlabial pad and a cover which overlaps a part of said main body, the cover having an end edge portion forming an unwrapping part for unwrapping said individual wrapping container, wherein said cover and said main body are lightly bonded each other at both ends in a perpendicular direction to an unwrapping direction of the individual wrapping container, and wherein the cover has an angle is made by a light-bonded line at both ends where the light-bonding is made and a non-bonded edge line where the light-bonding is not made, the angle which is made on other than the cover being an acute angle.
   According to such configuration, when that cover is picked up as it is for unwrapping the container after the cover overlapping the main body has been pulled up, the angle between the light-bonded line inside the pulling direction and non-bonded edge line becomes an acute angle and the force is concentrated to it, thereby making the unwrapping operation smooth. That is, according to such configuration, the inside of the both front and rear ends of the wrapping material forming the unwrapping portion is provided with an acute angle when unwrapping the unwrapping portion in the unwrapping direction so that it is possible for the wearer to concentrate the force of the fingers pinching the unwrapping portion in the unwrapping direction for unwrapping sufficiently on said acute angle section, thereby resulting in being easily peelable with the force in one direction (unwrapping direction), with unwrapping operation being rapid and easy.
(16) A individual wrapping container according to (14), comprising an angle formed between a light-bonded line at both end portions and a non-bonded non-bonded edge line of said cover, wherein the angle formed on other than the cover is an acute angle.

Because (16) has both configurations of said (14) and (15), in addition to the fact that the unwrapping part of the cover making an angle can be easily picked and the cover overlapping the main body can be easily picked up, when that cover is picked up as it is for unwrapping the container after the cover overlapping the main body has been pulled up, the angle between the light-bonded line inside the pulling direction and non-bonded edge line becomes an acute angle and the force is concentrated to it. Thereby, the unwrapping operation is made smooth.

### Brief Description of the Drawings

Fig. 1 shows a wrapping body relating to a preferred embodiment of the present invention.
Fig. 2 shows a cross-sectional view of the wrapping body in line A-A shown in Fig. 1.
Fig. 3 describes the sheet constituting the wrapping container of the wrapping body.
Fig. 4 shows the unwrapped state of the wrapping container.
Fig. 5 shows the interlabial pad contained in the wrapping body.
Fig. 6 is an unfolded view of the wrapping body.
Fig. 7 shows a process diagram showing the procedures of discarding the used interlabial pad.
Fig. 8 shows a variation example (rectangle type) of the profile of the cover of the wrapping body relating to a preferred embodiment according to the present invention.
Fig. 9 describes the unwrapping operation of the wrapping body relating to Fig. 8.
Fig. 10 shows a variation example (hypotenuse type) of the profile of the cover of the wrapping body relating to a preferred embodiment according to the present invention.
Fig. 11 shows a variation example (triangle type) of the profile of the cover of the wrapping body relating to a preferred embodiment according to the present invention.
Fig. 12 shows a variation example (gull wing type) of the profile of the cover of the wrapping body relating to a preferred embodiment according to the present invention.
Fig. 13 shows a variation example (sloped mountain type) of the profile of the cover of the wrapping body relating to a preferred embodiment according to the present invention.
Fig. 14 shows the state where an adhesive tape (sealing means) is provided on the main body of the wrapping body.
Fig. 15 describes the operation to unwrap the wrapping body relating to a preferred embodiment according to the present invention.
Fig. 16 describes the finger application region provided on the surface of the wrapping container in the wrapping body relating to relating to a preferred embodiment according to the present invention.
Fig. 17 describes the finger application region provided on the surface of the wrapping container in the wrapping body relating to a preferred embodiment according to the present invention.
Fig. 18 describes the finger application region (embossing) provided on the surface of the wrapping container in the wrapping body relating to a preferred embodiment according to the present invention.
Fig. 19 describes the devise to improve the easiness of unwrapping that is provided on the individual wrapping container relating to the present invention, showing a individual wrapping container in which a devise is made to improve the easiness of unwrapping relating to the present invention.
Fig. 20 describes the devise to improve the easiness of unwrapping that is provided on the individual wrapping container relating to the present invention, showing an comparative example in which no devise is made to improve the easiness of unwrapping relating to the present invention.
Fig. 21 shows how the conventional wrapping body of the interlabial pad is unwrapped.
Fig. 22 shows how the interlabial pad is contained in a conventional wrapping body.

### Best Mode of Carrying the Invention

Next, the embodiment of the present invention is described referring to drawings.

### [Basic Configuration]

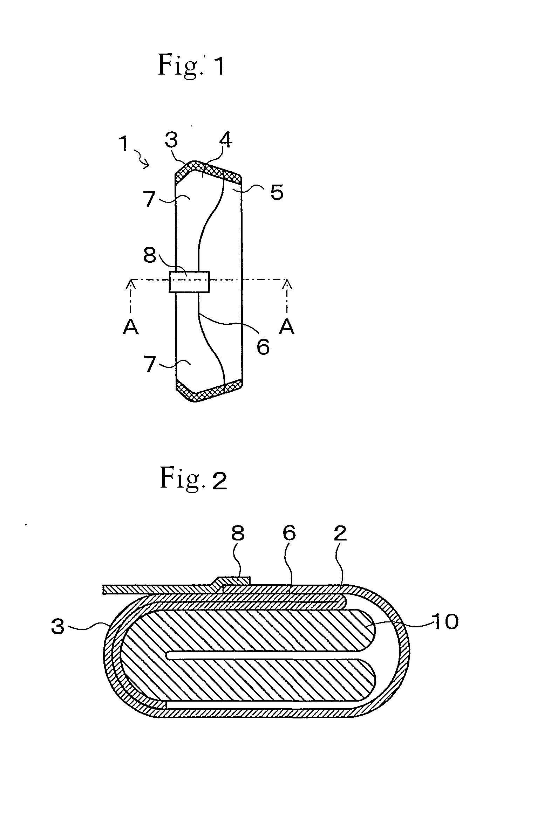

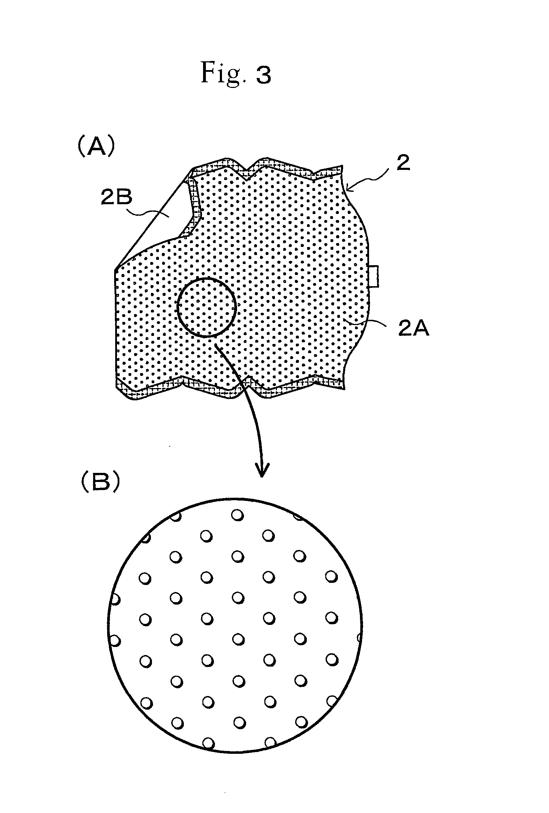

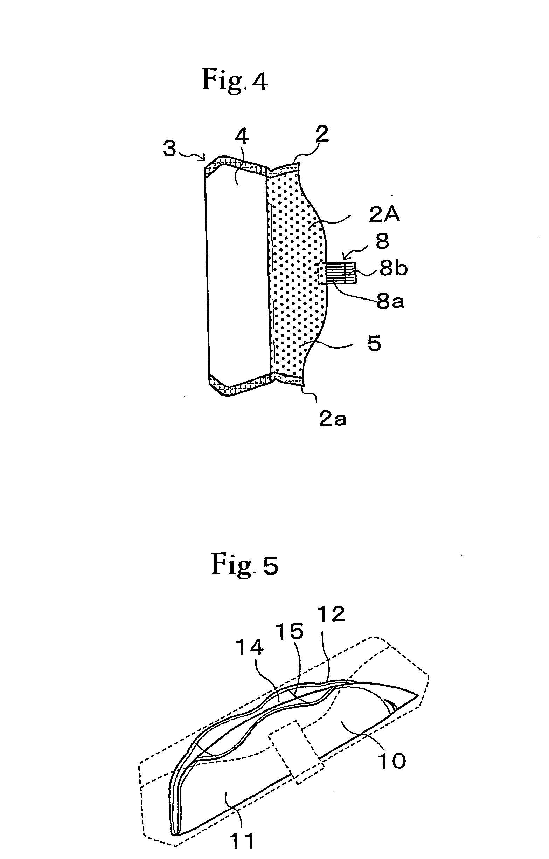

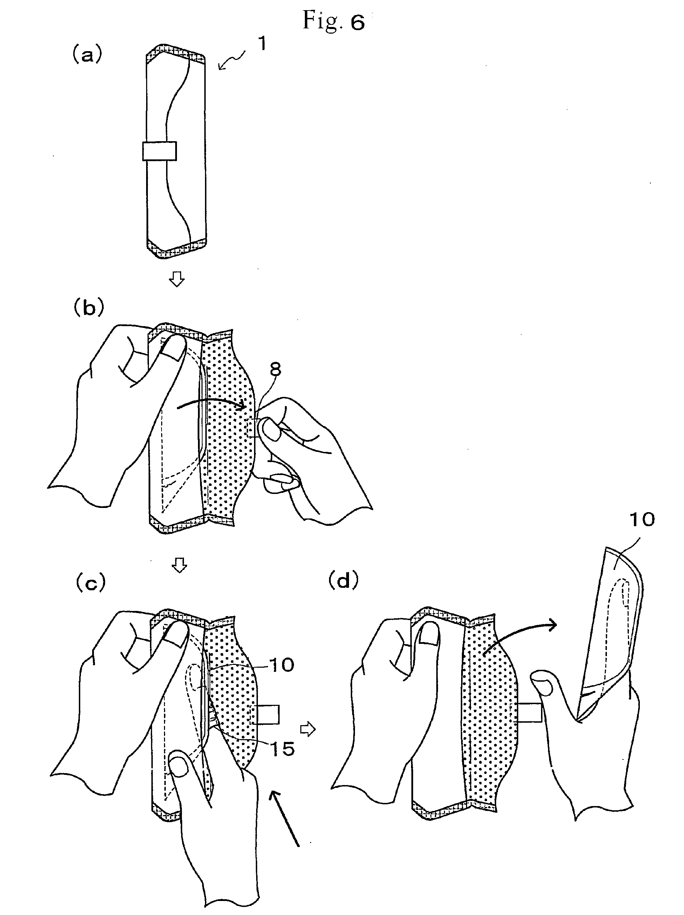

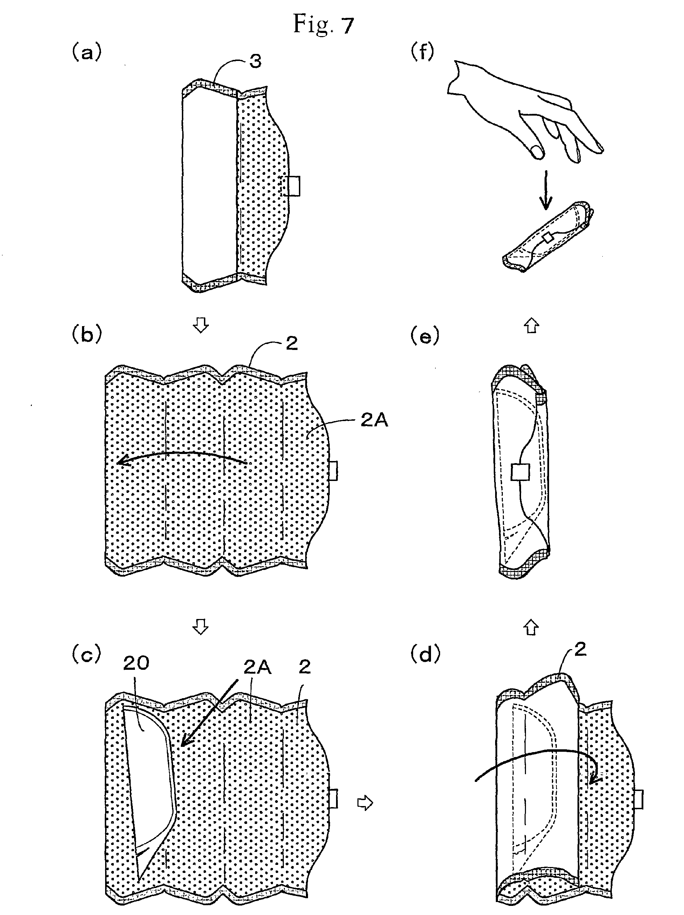

Firstly, the construction of the individual wrapping container (hereafter, called simply "wrapping container") and wrapping body in this embodiment are described. Fig. 1 shows the wrapping body 1 relating to this embodiment. Fig. 2 shows a cross-sectional view of the wrapping body 1 in line A-A shown in Fig. 1. Fig. 3 describes the sheet 2 constituting the wrapping container 3 of the wrapping body 1. Fig. 4 shows the unwrapped state of the wrapping container 3. Fig. 5 shows the interlabial pad 10 included in the wrapping body 1. Fig. 6 is an unfolded view of the wrapping body 1. Fig. 7 shows a process diagram showing the procedures of discarding the used interlabial pad 20.

### [Construction of wrapping body]

As shown in Fig. 1, the wrapping body 1 comprises a wrapping container 3 and an unused interlabial pad included therein (not shown). The wrapping container 3 has a main body 4 for containing an interlabial pad and a cover 5 covering this main body 4. The edge of the cover 5 is also used as an unwrapping part forming the unwrapping portion 6. That shape is curved so that a finger application region 7 is formed where fingers are applied for holding the main body 4 when a wearer unwraps it. Applying fingers on this region 7 to hold the wrapping body 1 prevents the wearer from simultaneously pressing the main body 4 and the cover.

### [Construction of Wrapping Container]

As shown in Fig. 2, the wrapping container 5 is formed by a wrapped sheet 2 to encapsulate the interlabial pad 10 folded with the longitudinal direction as a center line.

As shown in Fig. 3, this sheet 2 is flat on the one side 2B, however, is provided with fine convex on the other side 2A. It is possible to avoid the situation where the menstrual blood stains the inside of the wrapping container when the used interlabial pad is contained in the wrapping container 3 by a method wherein, as shown in Fig. 4, the wrapping container 3 is formed by folding the sheet 2 so that the surface 2A where such convex is provided is positioned inside.

As shown in Fig. 2, an unwrapping portion 6 is formed on the part where the sheet 2 overlaps. This unwrapping portion 6 is sealed by an adhesive tape 8, and as shown in Fig. 4, there are an adhesive portion 8a where re-pastable adhesive is applied and a dry edge 8b where no adhesive is applied on the under surface of the adhesive tape. This dry edge 8b is projecting from the base of the wrapping container 3 before the wrapping container 3 is unwrapped as shown in Fig. 1 so that the wearer can pick the adhesive tape 8 easily.

This adhesive tape 8 fixes the cover 5 and the main body 4 so that it is possible not to perform bonding for preventing the unwrapping of the unwrapping portion 6, however, it is also possible to enhance the bonding strength by providing a re-peelable bonding on the inside of the edge of the cover 4.

### [Interlabial pad]

As shown in Fig. 5, the interlabial pad 10 is folded to a half with the longitudinal direction as a center so that the opposite side to the body side 12 on which a mini-sheet piece 14 is attached will be inside. Therefore, it is possible to instantly view the mini-sheet piece 14 when the wrapping container 3 is unwrapped, a finger is inserted into the finger insertion opening 15 formed between this mini-sheet piece 14 and the opposite side face to the body side face 12 of the interlabial pad 10 to be able to instantly take out the interlabial pad 10 from the wrapping container 3. As a result, it is made possible to wear the pad between labia, maintaining the body side 11 of the interlabial pad 10 sanitary because the body side 11 of the interlabial pad 10 which directly contacts with the inner wall of labia when wearing does not contact with fingers without making wearing preparation troublesome.

Moreover, in this embodiment, the form of the interlabial pad 10 is a drop type, however, a wrapping container 3 is set in accordance with the semicircular shape wherein the interlabial pad 10 is folded along the longitudinal center line for wrapping. Therefore, a compactness for carrying the wrapping body 1 is enhanced, thereby further preventing that the interlabial pad 10 moves excessively in the wrapping container 3 due to the impact while carrying, resulting in the damage to the interlabial pad 10.

### [Unwrapped State]

When taking out the interlabial pad 10 by unwrapping the wrapping container 3, the adhesive tape 8 is picked with the right hand and pulled to outside for unwrapping while the wrapping body 1 shown in Fig. 6 (a) is held with the left hand as shown in Fig. 6 (b). Then, a finger is inserted into the finger insertion opening 15 of the interlabial pad 10 as shown in Fig. 6 (c) and the interlabial pad 10 is taken out as shown in Fig. 6 (d) by moving the right hand as it is. In this process, the fingers picking the wrapping body 1 simultaneously pick the wrapping container 3 and the interlabial pad 10 included therein so that it is prevented that the interlabial pad 10 springs out when unwrapping.

In this embodiment, the both sides of the main body section 5 is re-peelably bonded so as not to overlap the interlabial pad 10 included therein. Therefore, as shown in Fig. 7 (a), it is possible to completely unfold the wrapping container 3 into the one flat sheet 2 after an unused interlabial pad 10 has been taken out as shown in Fig. 7 (b). Here, fine convex portions are provided on one surface 2A of the sheet 2 so that, the used interlabial pad 20 is placed on this surface 2A as shown in Fig. 7 (c), it is wrapped in the sheet 2 as shown in Fig. 7 (d), and it is discarded as it is as shown in Fig. 7 (f), with a compact state as shown in Fig. 7 (e).

### [Other Form of Profile of Cover]

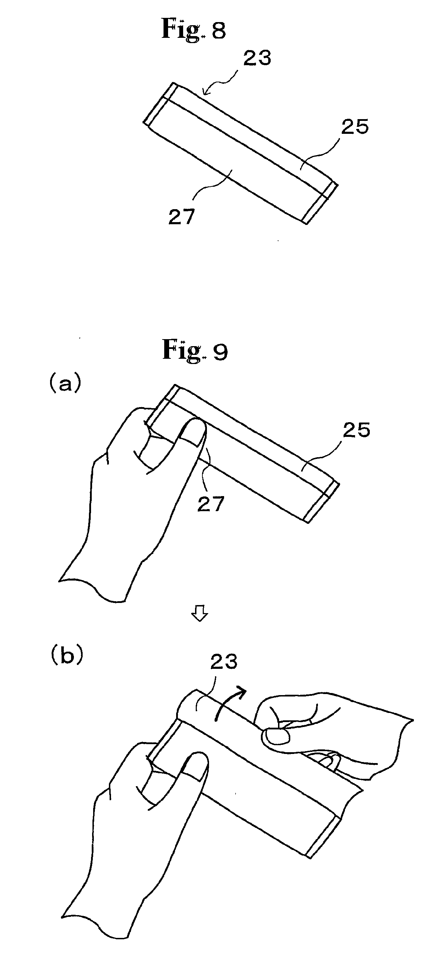

Nextly, other form of the profile of the cover of the wrapping container is described. Fig. 8 to Fig.12 show the variation example of the profile of the cover.

In the present invention, the profile of the edge of the cover can be shaped in such a way that the fingers of the wearer holding the main body are avoided so that it is possible to shape it in a form other than said M-shape. For example, as the wrapping container 23 shown in Fig. 8, it is possible to direct the wearer to hold the main body 25 by applying fingers on the finger application region under the unwrapping opening 26 as shown in Fig. 9 (a) when unwrapping the wrapping container 23 by making the cover 25 relatively short. Therefore, as shown in Fig. 9 (b), it is possible to smoothly conduct the unwrapping operation without re-holding the wrapping container 23 when unwrapping the wrapping container 23.

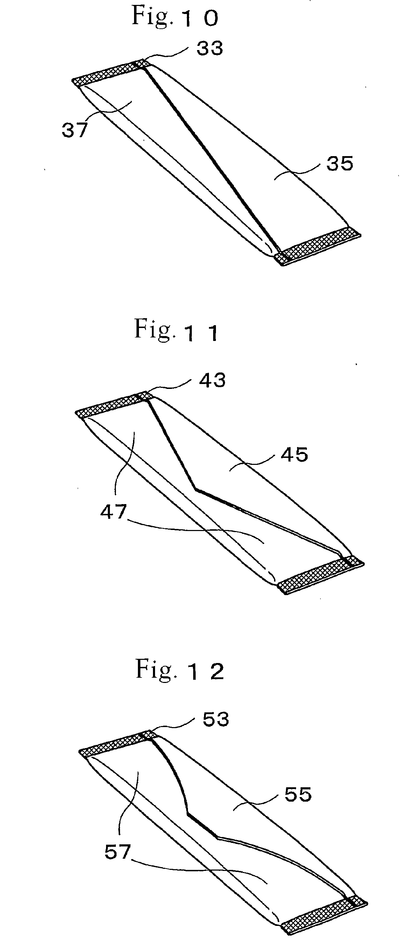

Moreover, as the wrapping container 33 shown in Fig. 10, it is possible to make the large exposure part of the main body 34 the finger application region 37 by making the profile of the cover 35 inclined.

Furthermore, as the wrapping container 43 shown in Fig. 11, it is possible to make the profile of the cover 45 V-shape. In this case, in contrast to Fig. 10, two finger application regions 47 are provided.

Moreover, as the wrapping container 53 shown in Fig. 12, it is possible to make the profile of the cover 55 "M" shape. In this case, two finger application regions 57 are provided.

### [Shape of Main Body]

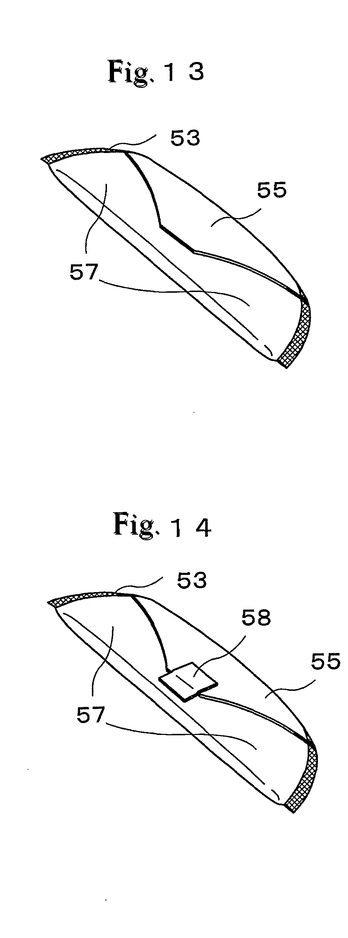

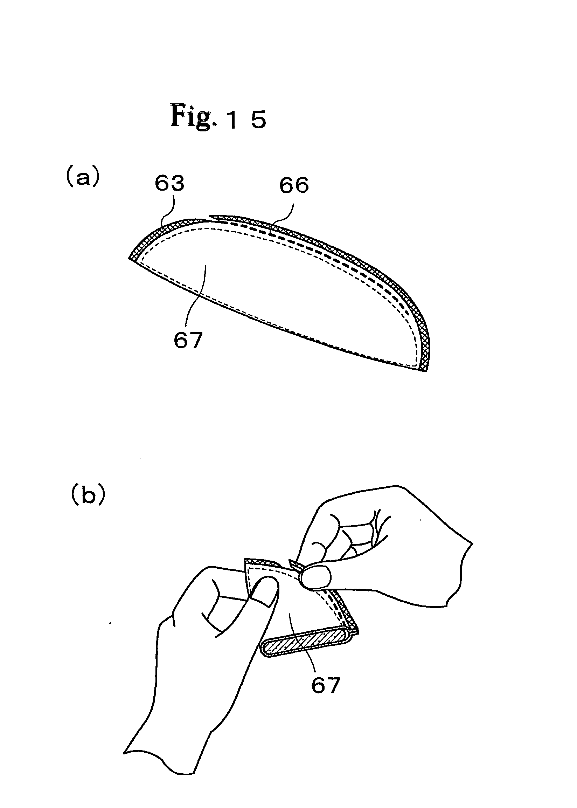

The shape of the main body is simply made a rectangle. In addition to that, it is possible to make the shape of the wrapping container 53 such a way that it gradually diminishes from base to the opening as shown in Fig. 13. It is possible to make the unwrapping operation easy by doing so. Moreover, as shown in Fig. 14, it is possible to provide an adhesive tape 58 on the cover 55.

### [Other Unwrapping Portion]

In the present invention, it is acceptable that the interlabial pad is contained in the wrapping container so that the interlabial pad is also held when the wrapping body is held in order to prevent the interlabial pad from springing out when the wrapping container is unwrapped. For example, as the wrapping container 63 shown in Fig. 15 (a), it is possible to make the perforation where a cut portion and non-cut portion are arranged alternatively around the unwrapping portion 66. In this case, as shown in Fig. 15 (b), a finger is applied to the finger application region 67, and the unwrapping portion 66 is unwrapped by tearing off one part of the wrapping container 63 along the perforation on the opposite side.

In the case where the unwrapping portion 66 is constituted by perforation, it is preferable that the cut portion is set to the range of 0.5 to 5 mm in length and maximum of 3 mm in width, and the non-cut portion is set to 0.5 to 3 mm in length. According to this structure, it is possible to direct the tearing direction of the wearer does not swerve from the unwrapping portion and prevent dirt from entering from the unwrapping portion to the inside of the individual wrapping body.

### [Indication of Finger Application Region]

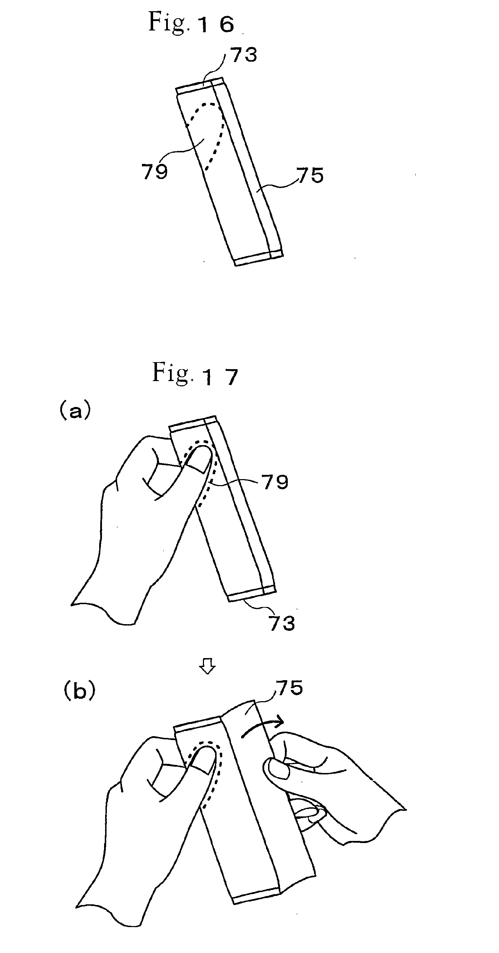

In the present invention, in order to show the wearer the position where fingers are applied to unwrap the wrapping portion, it is possible to indicate such position on the surface of the wrapping container. Fig. 16 and Fig. 17 show this indication.

A pattern 79 is provided in the wrapping container 73 shown in Fig. 16 in order to clearly indicate the position and direction where the fingers of the wearer are applied. Therefore, as shown in Fig. 17 (a), the wearer can hold the wrapping body 61 by applying the thumb in accordance with such pattern to unwrap the wrapping container 73 as shown in Fig. 17 (b).

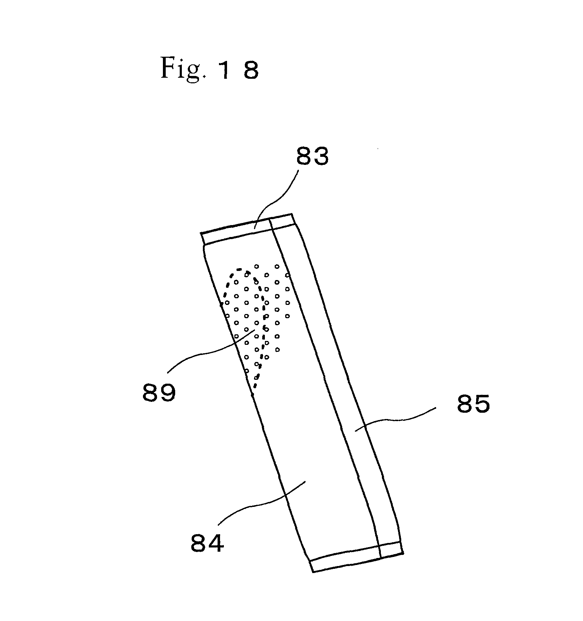

Moreover, as shown in the wrapping body 83 shown in Fig. 18, it is possible to clearly indicate the position and direction where the fingers of the wearer shall be applied by embossing 89 one part of the main body 84.

Moreover, the profile of the cover 75 and 85 is shaped in a straight line, it is also possible to make the profile of the cover a substantial M-shape and make a pattern or an embossing.

### [Devise to Improve Easiness of Unwrapping ]

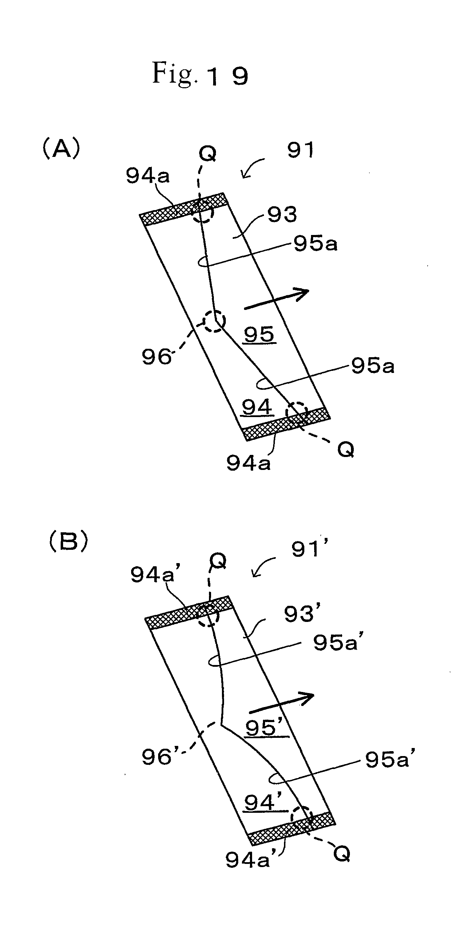

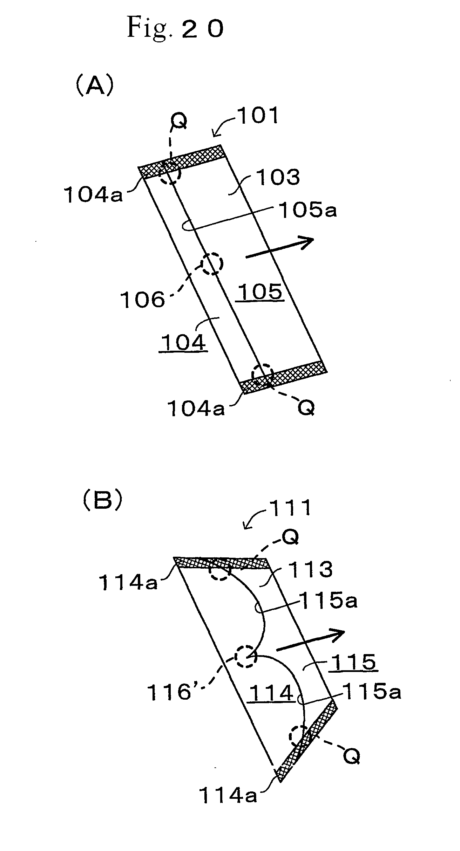

Fig. 19 and Fig. 20 describe the devise made to the individual wrapping container relating to the present invention, Fig. 19 shows the individual wrapping container on which a devise is made to improve the easiness of unwrapping. Fig. 19 shows the individual wrapping container relating to the present invention in which a devise is made to improve the easiness of unwrapping. Fig. 20 shows a comparative example. Moreover, in those Figures, the first digit "1" indicates the wrapping body, the first digit "3" indicates the wrapping container, the first digit "4" indicates the main body, the first digit "5" indicates the cover, and the first digit "6" indicates the unwrapping portion. Moreover, the end of the code "4a" indicates the lightly bonded line of the both ends where the cover "5" and the main body "4" are lightly bonded, and the end of the code "5a" indicates the non-bonding edge line where the cover "5" is not lightly bonded. Moreover, "Q" indicates the angle of the part where there is no cover "5" which is formed between the lightly bonded line "4a" of the both ends which is lightly bonded and the non-bonding edge line "5a" where the cover "5" is not lightly bonded.

First, as shown in Fig. 19 (A), in the individual wrapping container relating to the present invention, the part of the unwrapping portion 96 of the cover 95 in the individual wrapping container 93 is an [angle] with a specified angle. In this point, the unwrapping portion 106 of the individual wrapping container 103 in Fig. 20 (A) is a simple straight line in the part which corresponds to the unwrapping portion 96 relating to Fig. 19 (A). Comparing them, it is clear that the individual wrapping container 93 shown in Fig. 19 (A) is easier to be picked on the unwrapping portion 96 of the cover 95, and it is easier to pick up the cover 95 overlapping the main body 94.

Moreover, as shown in Fig. 19 (B), in the individual wrapping container relating to the present invention, the angle Q of the part is an acute angle where there is no cover 95 between the non-bonding edge line 95a of the cover 95 in the individual wrapping container 93 and the lightly bonded line 94a which is formed where the main body 94 of the individual wrapping container 93 and the cover 95 are lightly bonded. As shown in Fig. 19 (B), this "acute angle" state is maintained even if the non-bonding edge line 95a relating to Fig. 19(A) is curved to become a curved non-bonding edge line 95a'.

With this respect, the angle Q of the individual wrapping container 103 shown in Fig. 20 (A) is a right angle, and the angle Q of the individual wrapping container 113 shown in Fig. 20 (B) is an obtuse angle because the shape of the main body 114 is a trapezoid.

Here, comparing the individual wrapping containers 93 and 93' shown in Fig. 19 and the individual wrapping containers 103 and 113 shown in Fig. 20, the individual wrapping containers 93 and 93' shown in Fig. 19 indicates that the inside of the both front and rear ends of the wrapping material forming the unwrapping portion is an acute angle when the unwrapping portion is unwrapped in the unwrapping direction so that it is possible to sufficiently concentrate the force of the fingers in the unwrapping direction onto said acute angle part when the wearer pinches the unwrapping portion with fingers for unwrapping. As a result of this, it is possible to easily peel the individual wrapping container 93 and 93' against the force in one direction (unwrapping direction), thereby making the unwrapping operation quicker and easier.

### [Interlabial pad]

### <Shape>

The shape of the interlabial pad included in the wrapping body is not specially limited unless it is a shape that can be adopted to the labia without difficulty. However, it is preferable that it is substantially oval shape because the shape of the female labia is approximately symmetric. It is desirable that the shape is an elliptic type, a gourd-shape type, and drop-shape type.

Moreover, it is acceptable that it has a three-dimensional structure wherein there is a projection such as convex for closely contacting with the labia.

Moreover, it is acceptable that is has a mini-sheet piece forming a finger insertion opening on the garment side. Such interlabial pad makes it possible to wear the interlabial pad so as to close the ostium vaginae because it is possible to sense the position of ostium vaginae as a dent portion with sensitive finger by inserting a finger so that the fingerprint surface of the finger contacts with the rear side sheet, thereby preventing menstrual blood leak.

Moreover, as described above, it is preferable that, if the shape of the interlabial pad is an elliptic type, a gourd-shape type, and drop-shape type, the shape of the wrapping container is set in accordance with that. By doing so, the interlabial pad will be compact, enhancing the convenience while carrying.

The state of the interlabial pad included in the individual wrapping body is not specifically limited, however, considering the compactness while carrying and that a substantially oval interlabial product is closely contacted with the inner wall of labia symmetrically to prevent the leak of menstrual blood, it is preferable that, at least, it is folded along the longitudinal center line of the interlabial pad. Namely, the apparent longitudinal dimension of the interlabial pad included in the individual wrapping body is 80 to 150 mm and the substantially preferable apparent lateral dimension is the range of 10 to 30 mm.

### <Size>

The size of the interlabial pad should suitably be 50 to 200 mm in longitudinal dimension, preferably the range of 80 to 150 mm, and the apparent lateral dimension with respect to the body side should be 10 to 80 mm, preferably be the range of 20 to 40 mm.

If a projection such as convex is formed on the body side, the height of that projection can be set in the range of 5 to 30 mm, preferably 10 to 20 mm. If the dimension is less than this range, because the maximum absorption capacity of the interlabial pad is easily exceeded, there is a fear that the menstrual blood flows out. On the other hand, if the dimension is more than this range, the size of the napkin is exceeded, with a fear of poor wear feeling. Moreover, if the height range of the projection is low, it hampers sufficient close contact with the labia. On the other hand, if the dimension is higher than this range, it may give foreign feeling to the wearer.

The state of the interlabial pad included in the individual wrapping body is not specifically limited, however, considering the compactness while carrying and that an oval interlabial pad is closely contacted with the inner wall of labia symmetrically to prevent the leak of menstrual blood when wearing, it is preferable that, at least, it is folded along the longitudinal center line of the interlabial pad. It is possible to make the wearing process smooth by including it therein.

The preferable apparent longitudinal dimension of the interlabial pad included in the individual wrapping body is 80 to 150 mm and the substantially preferable apparent lateral dimension is the range of 10 to 30 mm.

### <Use>

The interlabial pad can be used for absorbing the excretion other than the menstrual blood discharged from the ostium vaginae other than the use in menstrual blood absorption, and it is also used for absorbing urine discharged from the urethral meatus, namely, for incontinence.

### [Constituent Material of Wrapping Container]

### <Sheet>

It is possible to use a known material for the sheet used for the wrapping container. For example, there are materials such as polyethylene, polypropylene, polyester, polyvinyl alcohol, poly-lactic acid, polybutylene succinate or nonwoven, paper, and laminated materials thereof in thickness of 15 -- 60 micron. Specifically, a film is generally used that is adjusted in the range of specific weight per unit area 15 to 30 g/m², with LDPE (Low-density polyethylene) as main constituent.

Considering the liquid interception characteristics, there is a suitable laminated material with nonwoven fabric or paper whose inner side is constructed by a film. Specifically, it is possible to use a composite wherein a film comprising mainly LDPE resin adjusted in the range of specific weight per unit area 5 to 20 g/m² is laminated on the one side of the composite nonwoven fabric comprising 6 to 10g/m² - 5 to 20g/m² - 6 to 10g/m² weight of spun bond-melt blown spun bond.

Moreover, if suitability of flushing is considered, it is possible to use poly-lactic acid which is biodegradable, polybutylene succinate, poly-lactic acid, polyisocyanate, nonwoven fabric made of starch, if water soluble, a film made of polyvynylalchohol, as well as a toilet paper. Specifically, there are a film made of polyvynylalchohol adjusted in the range of specific weight per unit area 5--10g/m² and a laminated body made of water-soluble paper adjusted in the range of specific weight per unit area 15--30g/m².

When used interlabial pad is discarded of, it is possible to use a sheet to wrap the interlabial pad for the discard of interlabial pad. In this case, it is preferable that the menstrual blood adhered or absorbed in the interlabial pad is prevented from leaking from the sheet, namely, the menstrual blood is prevented from staining on the finger when the wearer picks the sheet enveloping the used interlabial pad. Therefore, it is desirable to use a material with sufficient liquid stoppage such as a film made of polyethylene, polypropylene with thickness of about 15--60µ or laminated material wherein said film material is laminated on the nonwoven fabric or paper.

### <Shielding>

It is preferable that the sheet can shield the color of menstrual blood adhered or absorbed in the interlabial pad. To obtain such shielding effect, for example, ink made of pigment 5 to 40 weight %, resin 10 to 20 weight %, solvent 40 to 85 weight % is printed on the sheet for wrapping with photogravure or a sheet material which is made untransparent by mixing pigment 0.2 to 10 weight % with the raw material resin for the wrapping sheet is used. It is possible to dye a wrapping sheet. It is acceptable that the pigment or dyestuff is white or colored. For example, it is possible to use basic dye such as C.I. Basic violet 3; vat dye such as C.I. Vat blue 1; acid dye such as Blue color 1, Blue color 2, C.I. Acid red 51; direct dye such as C.I. Direct yellow 12, C.I. Direct orange 26, C.I. Direct violet 51, C.I. Direct Blue 1, C.I. Direct red 23; reactive dye such as C.I. Reactive orange 16, C.I. Reactive black 5, C.I. Reactive Blue 21, C.I. Reactive red 21; pigment dye such as Titanium oxide, Titanium yellow, Calcium carbonate, Carbon black, Ultramarine; organic dye such as Yellow 401, Orange 204, Blue 404, Red 201, C.I. Pigment yellow 14, C.I. Pigment green 7, C.I. Pigment violet 19, C.I. Pigment blue 27, C.I. Pigment red 166, independently or in combination as required.

It is preferable that the overlapping portion of the wrapping sheet forming the unwrapping portion can be repeeled and the mixing of foreign substance is prevented from entering. Such portion can be formed with a known technique wherein the overlapping portion of the wrapping sheet is covered each other by adhesive tape, a small amount of adhesive is applied along the overlapping direction, joint is made by embossing, and perforation is provided inside of the joint region through embossing.

### <Adhesive tape>

It is possible to use such known adhesive tape that a one side of a film layer is applied with adhesive or one side of a tissue paper is applied with adhesive if flushing is considered, without limitation.

### [Treatment Suppressing Number of Living Bacteria]

It is more preferable in sanitary aspects if, for the wrapping container relating to the present invention, a treatment to suppress the number of living bacteria is done, especially, on the part contacting with the interlabial pad. As a treatment to suppress the number of living bacteria, there are a sterilization or pasteurization treatment at production, and use of anti-bacteria material to the individual wrapping container for individual wrapping.

### [Constituent Material of Interlabial pad ]

### <Water-permeable Sheet>

A material which is liquid hydrophilic and does not irritate the skin is used for the water-permeable sheet arranged on the body side of the interlabial pad. As such material, there are a nonwoven fabric obtained from the fabrication method of Melt-blown, Spun bond, Point bond, Through air, Needle punch, wet-forming spun lace, and foam film is used independently or in combination.

As fiber sheet, there are a sheet made with a method wherein constituent of rayon, acetate, cotton, pulp or synthetic resin is composed independently or in combination to be provided independently or with a myelin construction.

Considering the liquid mobility from the inside surface of the labia, chemical stimulation due to the activation, and adhesion to the inner wall of labia, among such materials, spun lace nonwoven fabric is preferable which is manufactured with a method wherein rayon made of fineness 1.1--4.4 dtex and fiber length 7--51 mm are laminated in 40--80% with respect to total specific weight per unit area for the body side, and the rayon made of fineness 1.1--4.4 dtex and fiber length 7--51mm are laminated by mixing 6--18% PET with respect to total specific weight per unit area, and laminated so that the total specific weight per unit area of two layers is 20--60g/m², fibers are twined with water flow crossing to be dried, and the thickness is adjusted in the range of 0.13 to 0.50 mm for the garment side. In this instance, it is possible to maintain the adhesion with the inner wall of labia because the bulk is easily maintained by mixing PET in the clothing side even if the water permeable sheet is wet.

### <Absorbent body>

Pulp, chemical pulp, rayon, acetate, natural cotton, polymer absorbent body, fiber-form polymer absorbent body, synthetic fiber or mixture thereof can be used as the material used for the absorbent body included in the interlabial pad. It is possible to make the combined mixture a sheet with known technique such as press-fit through embossing or twining with needling, and adjust the amount as required, overlap or fold the sheet for adjustment.

It is possible to process the sheet-shaped material into a sheet or powder, without limitation in use.

It is preferable that the absorbent body can absorb and hold the liquid (body fluid), however, it is bulky and difficult to be deformed, less chemically stimulus, and has a higher flexibility to fit to labia. Specifically, there are nonwoven fabric with bulk of 2--10mm, preferably adjusted to 3--5mm which is manufactured with a method wherein the pulp selected from the fiber length 1--10mm is laminated by 50--150g/m² for the clothing side, and rayon selected from the fineness 1.1--4.4 dtex, and fiber length 20-51mm is laminated by 150--250g/m² with the mixing ratio with rayon 60--90% and natural cotton with 40--10%, and made to a sheet with dotted embossing for the body side. Therefore, it is made possible to easily move liquid from the body side to the clothing side, thereby enhancing the capacity of absorption and holding. Furthermore, mesh spun lace nonwoven fabric which is manufactured with a method wherein rayon made of fineness 11.1--4.4 dtex, fiber length 25--51mm is adjusted to specific weight per unit area 15--40g/m² is placed on the body side of said pulp layer so as to diffuse the liquid moved from the body side through the mesh spun lace and to guide the liquid to almost all region of the pulp layer, thereby making it possible to efficiently absorb more liquid.

### <Non-water Permeable Sheet>

It is possible to use the material for the non-water permeable sheet to be used for the interlabial pad that can prevent the menstrual blood held in the absorbent body from leaking outside the interlabial pad. Moreover, making the sheet moisture permeable material makes it possible to reduce the stuffiness while wearing, thereby making it possible to reduce the discomfort while wearing.

As such material, it is possible to use, for example, a sheet-shaped film made of synthetic resin, a porous film obtained by a method wherein inorganic filler is filled to be elongated, paper, a laminated material wherein a nonwoven fabric and a film are combined, a porous liquid shield sheet obtained by a method wherein capillary vessels with opening of 10--30% and diameter of 0.1--0.6 mm are arranged in the direction of the absorbent body.

Moreover, if flexibility not to hamper the wear feeling is considered, it is preferable to use a film obtained from the range of specific weight per unit area 15--30g/m², with main constituent of low density polyethylene (LDPE) resin with the density of 0.900--0.925g/m². More preferably, it is acceptable that, in order to reduce the fear that the interlabial pad drops from labia with higher friction while wearing between labia, when the non-water permeable sheet or pad or underwear are contacted, said film undergoes embossing and convex projection is provided to reduce the contacting ratio and friction value.

### <Adhesive>

It is possible to apply adhesive on the body side of the interlabial pad in order to strengthen the mating state between labia. As adhesive for adhering skin, there is a gel adhesive comprising aqueous polymer, cross-linking agent, plasticizer, and water. More specifically, there are gelatin, sodium poly acrylate, polyvynilalchohol, and carboxymethylcellulose as aqueous polymer; calcium chloride and a water-soluble metallic salt such as magnesium sulfate as cross-linking agent; glycerin, wax, paraffin as plasticizer.

In addition to that, it is possible to use pressure sensitive hot melt adhesive as adhesive to form the adhesion section. The main component of the pressure sensitive hot melt adhesive is synthetic rubber resin such as styrene-butadiene-styrene block copolymer (SBS), styrene-isoprene-styrene block copolymer (SIS), styrene-ethylene-butadiene-styrene block copolymer (SEBS), and styrene-ethylene-propylene-styrene block copolymer (SEPS), and the adhesive is obtained by melt-blending the adhesion adding agent such as terpene resin and rosin resin and the plasticizer such as wax therein.

Moreover, it is possible to use silicon resin adhesive. As silicon resin adhesive, there is a mixture with main component of silicon resin and fluorine resin obtained by a method wherein the cross-linking agent of metallic salt such as platinum, molybdenum, and antimony, and the plasticizer such as ester group wax, glycerin, machine oil are mixed.

There are many adhesives for forming the adhesive portion, however, considering the application stability, it is preferable to use pressure sensitive hot melt adhesive. As the pressure sensitive hot melt adhesive with higher application stability, there is one obtained by a method wherein 15--25 mass % of SEBS, 15--35 mass % of plasticizer, and 40--70 mass % of adhesion adding agent are melt-blended. For this pressure sensitive hot melt adhesive, it is possible to add oxidation preventing agent and fluorescent preventing agent in the range of 0.1--1.0 mass %.

### <Mini-sheet Piece>

It is possible to use the material similar to said water permeable sheet or non-water permeable sheet for the mini-sheet piece attached to provide the interlabial pad with the finger insertion opening, however, it is preferable to use the material having elongation characteristics or contracting characteristics at least in the lateral direction.

By using such material for the mini-sheet piece, it is possible to effectively use the interlabial pad relating to the present invention even if the finger size of the wearer is larger than the set finger insertion opening because the mini-sheet piece elongates at least in the width direction in response to the size of the finger.

As material having elasticity originally, there are styrene-butadiene-styrene block copolymer (SBS), styrene-isoprene-styrene block copolymer (SIS), synthetic rubber such as urethane, a film made of non-crystalline olefin group resin selected from the density of 0.88--0.900g/m³, a porous foam film, and a net. Moreover, it is possible to use woven cloth or cloth wherein twined filament made of synthetic rubber is woven in the cloth. Moreover, it is possible to use spun bond nonwoven fabric mainly made of synthetic rubber and melt blown nonwoven fabric, and foaming sheet.

Considering the flexible feel while wearing, there is a porous foam film made of SEBS, with adjusted thickness of 15--40 micron, porous area being 0.28--1.77m m², and rate of hole area being 40--70%.

As nonwoven fabric, there are a spun lace nonwoven fabric which is made of composite synthetic fiber such as PE/PP, PE/PET, PP/PP, etc. having thermal contraction characteristics, with core component having high melting point and myelin comprising low melting point component and wherein the fibers are twined by water flow pressure, a shrink type nonwoven fabric wherein re-heating hot wind process is conducted to promote the shrink of the fiber, and a elongation type spun bond wherein the tentering is forcibly done in the lateral direction after the sheet is manufactured from continuous long fibers with thermal seal.

More specifically, as a suitable material with flexible and rich drape feeling, there is a shrink type nonwoven fabric made of composite synthetic fiber such as PE/PP, PE/PET, PP/PP, etc. having thermal contraction characteristics, with core component having high melting point and myelin comprising low melting point component and with specific weight per unit area adjusted in the range of 10--60g/m². Furthermore, it is possible to use laminated product of said material.

In the case where non-elongation material is used by adding elongation characteristics, there are through-air nonwoven fabric with rich bulky feeling which is made of composite synthetic fiber such as PE/PP, PE/PET, PP/PP, etc. having thermal contraction characteristics, with core component having high melting point and myelin comprising low melting point component and underwent hot air processing, spun lace nonwoven fabric whose fibers are twined by water flow pressure, spun bond nonwoven fabric wherein continuous fibers are laminated to be a sheet, needle punch nonwoven fabric wherein fibers are twined by needles, SMS nonwoven fabric wherein spun bond and melt blown are laminated in multiple layers to be a sheet, a porous foam film, a material wherein a film mainly made of PE resin is combined independently or in combination.

Moreover, it is also possible to add elongation characteristics by colligate processing wherein said material is mated between the male and female metal molds, and a shape is pressed through heat, temperature, and pressure. More specifically, there is a material wherein through-air nonwoven fabric mainly made of composite synthetic fibers adjusted in the range of 1.1--4.4 dtex in thickness and specific weight per unit area 10--60g/m² is colligated in lateral direction elongatably. Such colligate processing is preferable that the array of male and female metal molds is provided to allow elongation of, at least, more than 10%, preferably elongatable in the range of 20--50%, more preferably, the load at 30% elongation having behavior in the range of 0.01--0.05N/25mm (Testing condition: Speed 100mm/moin, Chuck interval 100mm with Tensilon tensile tester). Moreover, it is possible to use cut line and circular cut as other method to add elongation.

### [Configuration of Wrapping container and Interlabial Pad provided with Biodegradability, Water Dispersibility, and Water solubility]

### <Wrapping Container>

It is also possible to use a biodegradable material, and/or water-soluble and/or water dispersible material for the constituent material of the wrapping container constituting the wrapping body relating to the present invention. In the case where the material for the wrapping container is water soluble material and/or water dispersible material, the wearer can be released from the troublesomeness of discarding the wrapping container and reduce the debris in the toilet because it is possible to flush it into the toilet as it is wrapped if the used interlabial pad included in that material is also water soluble or water dispersible.

As an example of such wrapping container, there are a composite material wherein a tissue adjusted to specific weight per unit area 15--40g/m² and polyvynilalchohol with specific weight per unit area of 20--50g/m² are laminated and silicon is applied on the polyvynilalchohol side in the range of 0.5--1µm, and spun bond nonwoven fabric mainly made of poly-lactic acid fiber and adjusted to specific weight per unit area 15--40g/m².

Moreover, in this Specification, "biodegradability" means that a substance is decomposed into gas such as carbon dioxide or methane, water, and biomass under anaerobic or aerobic condition according to the natural process under the existence of bacteria represented by actinomycetes and other microbes, and also means that the biodegradability (biodegradable rate and biodegradable degree) of the substance equals to a material naturally generated such as fallen leaves or a synthetic polymer generally recognized having the same biodegradability under the same environment. "Water dispersibility" is the same meaning as water degradability, and means character that, even though there is no effect with the limited amount of water (menstrual blood) when using, but in large amount of water or in water flow, the fibers can be easily dispersed into small pieces in the degree that it does not clog at least a general toilet piping. "Water soluble" means the character that, even though there is no effect with the limited amount of water (menstrual blood) when using, but it is solved in large amount of water or water flow.

### <Configuration of Inter-labia Pad>

It is preferable that the interlabial pad included in said wrapping container is biodegradable/water soluble/water dispersible. By doing so, it is possible to easily and cleanly disperse of the used interlabial pad because the interlabial pad can be flushed into the toilet as it is. Because it is made possible to flush the interlabial pad with the wrapping container, it is also possible to reduce the trash in the toilet furthermore.

### [Water Permeable Sheet]

As a material for water permeable sheet, it is possible to use wet type spun lace nonwoven fabric whose fiber length can be selected from the range of 1--15mm, other than spun lace nonwoven fabric. As other material, it is possible to use a biodegradable resin by means of hydrolysis such as poly-lactic acid and plybutylene succinate. For example, there are melt blown nonwoven fabric made of poly-lactic acid adjusted in the range of specific weight per unit area 20--60g/m², and spun bond nonwoven fabric adjusted in the range 15-30 g/m² and in the range of 1.1--3.3dtex in fiber length. Moreover, it is acceptable whether the nonwoven fabric material is bored or not.

As other material, it is possible to use independently synthetic fiber or by adjusting the cane which is a continuous fiber of laminated body in the range of specific weight per unit area 50--300g/m² and separating fibers each other.

### [Absorbent body]

As a material used for the absorbent body, it is possible to use nonwoven fabric sheet obtained by needling. Moreover, considering the biodegradability of the polymer absorber material, it is preferable to use carboxymethylcellulose fiber.

### [Water Impermeable Sheet]

As a material used for the non-water permeable sheet, it is possible to use a PVA film, a film sheet wherein water repellent treatment is partly done on one side or both sides of the PVA film with silicon, a PVA film mixed with silicon, a starch film, a film made of biodegradable resin by means of hydrolysis such as poly-lactic acid or plybutylene succinate, and laminated paper with tissue, etc. It is acceptable to color the material by mixing inorganic pigment in the range of 0.1--5% as required.

If it is considered that leak prevention is maintained under over-moisture and that septic tank is not excessively loaded, it is suitable to use a laminated paper which is manufactured by a method wherein a film made of poly-lactic acid is laminated with a tissue selected from the range of specific weight per unit area 15--20g/m² in the range of thickness of 10-20 micron, and further the pasting area rate at lamination is set in the range of 5--40%.

### [Mini-Sheet Piece]

As a material for the mini-sheet piece, there are a film made of biodegradable material such as poly-lactic acid and plybutylene succinate, spun bond nonwoven fabric, melt blown nonwoven fabric, or a film made of water soluble material such as PVA, CMC, a water dispersive tissue made of nonwoven fabric as well as cellulose fiber, and recycle cellulose fiber, and spun lace nonwoven fabric.

It is preferable to use spun bond nonwoven fabric mainly made of biodegradable material or melt blown nonwoven fabric, a sheet adjusted in the range of 0.1--3.3 dtex in thickness, in the range of specific weight per unit area 15--40g/m² and obtained by said mechanical colligate process.

### [Method of Bonding]

As a method of bonding, it is possible to use a bonding method such as adhesion by means of polyvynilalchohol having water solubility or water swelling characteristics, heat seal, or junction by means of hydrogen bonding independently or in combination as required. Moreover, in order to lightly bond the material, for example, in the part of cover and main body, the amount of the adhesive or state of the heat seal is set as necessary so that it is possible to easily peel the part by female operation.

### [Possibility of Industrial Use]

As described above, according to the interlabial pad individual wrapping container relating to the present invention, it is possible to easily conduct the operation wherein the individual wrapping container wrapping the unused interlabial pad compactly is unwrapped. Therefore, it is possible to wear the interlabial pad sanitarily and smoothly.

## Claims

1. An individual wrapping container for wrapping individually an interlabial pad to be held between labia, comprising:
a main body containing said interlabial pad
and a cover that covers a part of said main body, the cover forming an unwrapping portion at an end edge portion to be unwrapped to open the individual wrapping container;
wherein said cover is shaped to have a cut off portion at a place where said individual wrapping container is held when the individual wrapping container is opened, and
wherein the individual wrapping container has predetermined region where a finger of a wearer is applied.

2. An individual wrapping container according to claim 1, **characterized in that** said end edge portion of said cover is inflected or inclined to a bottom side of the individual wrapping container.

3. An individual wrapping container for wrapping individually an interlabial pad to be held between labia, comprising:
a main body containing said interlabial pad and
a cover that covers a part of said main body, the cover forming an unwrapped portion at an end edge portion to be unwrapped to open the individually wrapping container;
wherein a surface of said main body has an instruction to show where said individual wrapping container is held when opening the individual wrapping container is unwrapped.

4. An individual wrapping container according to claim 3, **characterized in that** said instruction is made visual or touchable.

5. An individual wrapping container for wrapping individually an interlabial pad to be held between labia, comprising:
a main body containing said interlabial pad and
a cover which overlaps on a part of the main body, the cover having an edge portion forming an unwrapping portion to unwrap said individual wrapping container;
wherein the main body has a fine projection at least on an internal surface of said main body.

6. An individual wrapping container according to any one of claims 1 - 5,
wherein said individual wrapping container comprises a container on which a series of sheets for wrapping is wound, and
wherein said cover comprises an overlapping portion that is formed by overlapping both ends of a wrapping sheet and a vicinity of said both ends, and
wherein said unwrapping portion comprises one end of said wrapping sheet, and
wherein said individual wrapping container is unwrapped by unfolding said overlapping portion from said unwrapping portion.

7. An individual wrapping container according to claim 6 comprising:
an unwrapping portion for sealing the interlabial pad, and
a bottom line on the opposite side;
wherein the dimension of the main body becomes smaller as it proceeds from said bottom line to said unwrapping portion.

8. An individual wrapping container according to any one of claims 1 - 7,
wherein said individual wrapping container is provided with a re-sealable sealing means on a portion where said cover and said main body are brought into contact.

9. An individual wrapping container according to claim 8, **characterized in that** said sealing means has a dry edge and that said dry edge projects from the bottom side of said individual wrapping container.

10. A wrapping body, comprising an interlabial pad to be held between labia and an individual wrapping container for individual wrapping said interlabial pad, wherein the interlabial pad wrapped in said individual wrapping container constitutes the wrapping body, and
wherein the interlabial pad in said individual wrapping container is arranged on a position where at least one part of said interlabial pad is held at the same time when said individual wrapping container is held.

11. An individual wrapping body according to claim 10, wherein said individual wrapping container is the individual wrapping container as recited in any one of claims 1 - 9.

12. A wrapping body according to claim 11,
wherein said interlabial pad and said individual wrapping container have a longitudinal direction and a lateral direction, and substantially form an elongated shape in the longitudinal direction,
wherein a dimension in the longitudinal direction of said individual wrapping container is in a range of 105 -- 130% of a dimension in the longitudinal direction of said interlabial pad.

13. A wrapping body according to claim 10 or 11, wherein the dimension in said lateral direction of said individual wrapping container is set in a range of 105 -- 130% of the dimension in the lateral direction of said interlabial pad.

14. An individual wrapping container for individually wrapping an interlabial pad to be held between labia, comprising:
a main body including said interlabial pad and
a cover which overlaps a part of said main body, the cover having an end edge portion forming an unwrapping position to unwrap the individual wrapping container,
wherein said cover and said main body are lightly bonded each other at both end portions in a perpendicular direction to an unwrapping direction of the individual wrapping container, and
wherein said cover has an angular shape extending toward both sides of said main body from one part of said unwrapping part as a vertex.

15. An individual wrapping container for individually wrapping an interlabial pad to be held between labia, comprising:
a main body containing said interlabial pad and
a cover which overlaps a part of said main body, the cover having an end edge portion forming an unwrapping part for unwrapping said individual wrapping container, and
wherein said cover and said main body are lightly bonded each other at both ends in a perpendicular direction to an unwrapping direction of the individual wrapping container, and
wherein an angle is made by a light-bonded line at both ends where the light-bonding is made and a non-bonded edge line where the light-bonding is not made, the angle which is made on a place other than the cover being an acute angle.

16. An individual wrapping container according to claim 14, comprising:
an angle formed between a lightly-bonded line at both end portions and
a non-bonded edge line of said cover,
wherein the angle formed on a place other than the cover is an acute angle.
